# EUROPEAN PATENT APPLICATION

(11) **EP 3 135 296 A1**
(43) Date of publication of application: **01.03.2017**
(21) Application number: 15755876.8
(22) Date of filing: 26.02.2015
(51) Int. Cl.: A61K 45/00, A61K 38/00, A61K 39/395, A61K 47/34, A61K 47/42, A61K 47/62, A61P 5/00, A61P 19/00, A61P 43/00, A61K 45/06

(54) **MEDICINE AGAINST GROWTH IMPAIRMENT INDUCED BY ADMINISTRATION OF STEROID**

(30) Priority: 27.02.2014 JP 2014036780
(71) Applicant: Daiichi Sankyo Company, Limited, Tokyo 103-8426 (JP)
(72) Inventor: MOROZUMI, Naomi, Kobe-shi Hyogo 650-0047 (JP)
(74) Representative: Arends, William Gerrit
(86) International application number: PCT/JP2015/055650
(87) International publication number: WO 2015/129812

(57) **Abstract**

Provided is a medicine for reducing and/or ameliorating growth failure induced by the administration of a steroid during a growth period, the medicine containing at least one type of natriuretic peptide receptor B (NPR-B) agonist as an active ingredient.

SEQUENCE LISTING

<110>  DAIICHISANKYO COMPANY, LIMITED

<120>  An agent against growth-disorder derived by steroides.

<130>  FP1511

<150>  JP2014-036780
<151>  2014-02-27

<160>  15

<170>  PatentIn version 3.5

<210>  1
<211>  22
<212>  PRT
<213>  mammalian/homo sapience

<400>  1

Gly Leu Ser Lys Gly Cys Phe Gly Leu Lys Leu Asp Arg Ile Gly Ser
1               5                   10                  15

Met Ser Gly Leu Gly Cys
            20

<210>  2
<211>  53
<212>  PRT
<213>  mammalian

<400>  2

Asp Leu Arg Val Asp Thr Lys Ser Arg Ala Ala Trp Ala Arg Leu Leu
1               5                   10                  15

Gln Glu His Pro Asn Ala Arg Lys Tyr Lys Gly Ala Asn Lys Lys Gly
            20                  25                  30

Leu Ser Lys Gly Cys Phe Gly Leu Lys Leu Asp Arg Ile Gly Ser Met
        35                  40                  45

Ser Gly Leu Gly Cys
    50

<210>  3
<211>  22
<212>  PRT
<213>  Gallus gallus

<400>  3

Gly Leu Ser Arg Ser Cys Phe Gly Val Lys Leu Asp Arg Ile Gly Ser
1               5                   10                  15

Met Ser Gly Leu Gly Cys
            20

<210>  4
<211>  22
<212>  PRT
<213>  Rana sp.

<400>  4

Gly Tyr Ser Arg Gly Cys Phe Gly Val Lys Leu Asp Arg Ile Gly Ala
1               5                   10                  15

Phe Ser Gly Leu Gly Cys
            20

<210>  5
<211>  17
<212>  PRT
<213>  Artificial

<220>
<223>  A ring structure portion of CNP

<220>
<221>  MISC_FEATURE
<222>  (11)..(12)
<223>  the amino acid could be Ser or Ala on position 11 and Met, Phe or
       Glu

 on position 12

<400>  5

Cys Phe Gly Leu Lys Leu Asp Arg Ile Gly Xaa Xaa Ser Gly Leu Gly
1               5                   10                  15

Cys

<210>  6
<211>  34
<212>  PRT
<213>  Artificial

<220>
<223>  Chimeric CNP A of US2010-305031 and WO2009/142307

<400>  6

Arg Pro Gln Leu Lys Ala Pro Pro Lys Lys Ser Glu Lys Arg Gln Gln
1               5                   10                  15

Val Cys Phe Gly Leu Lys Leu Asp Arg Ile Gly Ser Met Ser Gly Leu
            20                  25                  30

Gly Cys

<210> 7
<211> 34
<212> PRT
<213> Artificial

<220>
<223> Chimeric CNP B (SEQ ID NO;110) of US2010-305031 and WO2009/142307

<400> 7

Cys Phe Gly Leu Lys Leu Asp Arg Ile Gly Ser Met Ser Gly Leu Gly
1               5                   10                  15

Cys Val Gln Gln Arg Lys Glu Ser Lys Lys Pro Pro Ala Lys Leu Gln
            20                  25                  30

Pro Arg

<210> 8
<211> 39
<212> PRT
<213> Artificial

<220>
<223> Chimeric CNP D (SEQ ID NO;125) of US2010-305031 and WO2009/142307

<400> 8

Gly Leu Ser Lys Gly Cys Phe Gly Leu Lys Leu Asp Arg Ile Gly Ser
1               5                   10                  15

Met Ser Gly Leu Gly Cys Val Gln Gln Arg Lys Glu Ser Lys Lys Pro
            20                  25                  30

Pro Ala Lys Leu Gln Pro Arg
        35

<210> 9
<211> 51
<212> PRT
<213> Artificial

<220>
<223> Chimeric CNP E (SEQ ID NO;126) of US2010-305031 and WO2009/142307

<400> 9

Val Gln Gln Arg Lys Glu Ser Lys Lys Pro Pro Ala Lys Leu Gln Pro
1               5                   10                  15

Arg Cys Phe Gly Leu Lys Leu Asp Arg Ile Gly Ser Met Ser Gly Leu
            20                  25                  30

Gly Cys Val Gln Gln Arg Lys Glu Ser Lys Lys Pro Pro Ala Lys Leu
        35                  40                  45

Gln Pro Arg
    50

<210>  10
<211>  51
<212>  PRT
<213>  Artificial

<220>
<223>  Chimeric CNP F (SEQ ID NO;127) of US2010-305031 and WO2009/142307

<400>  10

Arg Pro Gln Leu Lys Ala Pro Pro Lys Lys Ser Glu Lys Arg Gln Gln
1               5                   10                  15

Val Cys Phe Gly Leu Lys Leu Asp Arg Ile Gly Ser Met Ser Gly Leu
            20                  25                  30

Gly Cys Val Gln Gln Arg Lys Glu Ser Lys Lys Pro Pro Ala Lys Leu
        35                  40                  45

Gln Pro Arg
    50

<210>  11
<211>  34
<212>  PRT
<213>  Artificial

<220>
<223>  Chimeric CNP G (SEQ ID NO;128) of US2010-305031 and WO2009/142307

<400>  11

Val Gln Gln Arg Lys Glu Ser Lys Lys Pro Pro Ala Lys Leu Gln Pro
1               5                   10                  15

Arg Cys Phe Gly Leu Lys Leu Asp Arg Ile Gly Ser Met Ser Gly Leu
            20                  25                  30

Gly Cys

<210>  12
<211>  34

<212>  PRT
<213>  Artificial

<220>
<223>  Chimeric CNP I (SEQ ID NO;130) of US2010-305031 and WO2009/142307


<220>
<221>  Amidation
<222>  (34)..(34)
<223>  The Arg residue on Cterminal is amidated

<220>
<221>  Amidation
<222>  (34)..(34)
<223>  The 34Arg residue on Cterminal end is amidated

<400>  12

Cys Phe Gly Leu Lys Leu Asp Arg Ile Gly Ser Met Ser Gly Leu Gly
1               5                   10                  15


Cys Val Gln Gln Arg Lys Glu Ser Lys Lys Pro Pro Ala Lys Leu Gln
            20                  25                  30


Pro Arg




<210>  13
<211>  39
<212>  PRT
<213>  Artificial

<220>
<223>  Chimeric CNP J (SEQ ID NO;157) of US2010-305031 and
       WO2009/142307, and CNP derivative A of current invention.

<400>  13

Gly Leu Ser Lys Gly Cys Phe Gly Leu Lys Leu Asp Arg Ile Gly Ser
1               5                   10                  15


Met Ser Gly Leu Gly Cys Val Gln Gln Arg Lys Asp Ser Lys Lys Pro
            20                  25                  30


Pro Ala Lys Leu Gln Pro Arg
        35


<210>  14
<211>  36
<212>  PRT
<213>  Artificial

<220>
<223>  Chimeric CNP K (SEQ ID NO;158) of US2010-305031 and
       WO2009/142C307

<400>  14

Cys Phe Gly Leu Lys Leu Asp Arg Ile Gly Ser Met Ser Gly Leu Gly
1               5                   10                  15

Cys Ala Gly Ser Val Asp His Lys Gly Lys Gln Arg Lys Val Val Asp
            20                  25                  30

His Pro Lys Arg
        35

<210>  15
<211>  39
<212>  PRT
<213>  Artificial Sequence

<220>
<223>  CNP derivative B of current invention, which disclosed in Am. J. Hum.
Gent.(2012),
       91(6), 1108-1114

<400>  15

Pro Gly Gln Glu His Pro Asn Ala Arg Lys Tyr Lys Gly Ala Asn Lys
1               5                   10                  15

Lys Gly Leu Ser Lys Gly Cys Phe Gly Leu Lys Leu Asp Arg Ile Gly
            20                  25                  30

Ser Met Ser Gly Leu Gly Cys
        35

## Description

### Technical Field

The present invention relates to a medicine for reducing and/or ameliorating growth failure induced by the administration of a steroid, the medicine containing a natriuretic peptide receptor B (NPR-B) agonist such as a C-type natriuretic peptide (CNP) or a CNP derivative as an active ingredient.

### Background Art

"Steroids" is a generic term for drugs containing a steroid hormone or a chemically synthesized modified compound thereof, and typically means pharmaceuticals containing a glucocorticoid or a chemically synthesized modified compound thereof. Steroids have pharmacological actions such as an anti-inflammatory action, an immunosuppressive action, a lymphocytotoxic action, a vasoconstriction action, and a bronchodilation action, and are known to have dramatic effects on diverse diseases. Meanwhile, the risk of the appearance of serious side effects has been reported for therapies with steroids, and typical side effects include development or worsening of infectious diseases by excessive immunosuppressive action, Cushing syndrome, dysadrenocorticism, diabetes, osteoporosis and peptic ulcer (Non Patent Literature 1, Non Patent Literature 2 and so on). Also indicated is the risk of worsening the condition of the primary disease to be treated (rebound) and developing a so-called "steroid withdrawal syndrome" involving nausea, headache, malaise, fall in blood pressure or the like if the patient neglects the administration instructions of the physician for fear of the appearance of side effects by the steroid therapy as described above (Non Patent Literature 3).

In infants and young children, in addition to the above, long-term use of a steroid is associated with the risk of the occurrence of growth failure. Long-term therapy with a steroid in an infant or young child results in the prevention of the generation and secretion of growth hormone and insulin-like growth factor (IGF-1) by glucocorticoids that are excessive in the body, or results in the deterioration in sensitivity to such hormones. This causes a condition whereby the normal growth of the infant or young child is suppressed, namely "growth failure", and can result in the diagnosis of so-called "dwarfism" which is a condition in which height is significantly lower in comparison with the standard growth curve of children of the same age (Non Patent Literature 4). Examples of diseases for which a relatively long-term therapy with a steroid is required in infants and young children include asthma, atopic dermatitis, systemic lupus erythematosus, rheumatic fever, juvenile idiopathic arthritis, ulcerative colitis, Crohn's disease, myasthenia gravis, Guillain-Barre syndrome, pulmonary hemosiderosis, infantile IgA nephropathia, idiopathic thrombocytopenic purpura, autoimmune hemolytic anemia, aplastic anemia, acute lymphocytic leukemia, and congenital adrenal hyperplasia. At least some of these are such serious diseases that it is difficult to stop therapy with a steroid, and death can be caused if the diseases become worse. Therefore, there is a demand to minimize the appearance of various side effects while continuing to use a steroid within the range that attains the intended effect to a sufficient degree.

While growth failure induced by the administration of a steroid is at least partly recovered by subsequently ceasing or reducing administration (Non Patent Literature 5), the growth period of an infant or young child is limited, and once the epiphyseal growth plate has closed as the child reaches puberty, further growth is not expected irrespective of use of a steroid. Therefore, in subjects for whom continuous or continual therapy with a steroid is required, and for whom ceasing of administration or reduction of dosage cannot be made in the long term due to a serious, fatal disease in his/her childhood, sufficient growth is not expected through his/her life, and the possibility of suffering from dwarfism arises.

From the above, if there were a drug capable of reducing and/or ameliorating growth failure occurring due to steroid therapy without reducing the therapeutic effect of the steroid, and without worsening the side effects other than the growth failure in an infant or young child in need of therapy with the steroid, or a drug capable of promoting the growth of an infant or young child suffering from dwarfism caused by steroid therapy, and treating the dwarfism, improvement of QOL through the life of the subject would be expected. Further, reducing the risk of growth failure allows therapy of the primary disease with an adequate amount of steroid, and this can lead to an improvement in the outcome of the primary disease.

Presently, growth hormone, IGF-1 or the like is used as a therapeutic agent for dwarfism caused by certain diseases. These drugs are very expensive, and face the fear of the appearance of various side effects (Non Patent Literature 6). Therefore, prophylactic or therapeutic administration of these drugs for growth failure induced by the administration of a steroid is not recommended in consideration of the risk and benefit.

C-type natriuretic peptide (C-Type Natriuretic Peptide: hereinafter referred to as CNP) is an agonist of NPR-B that expresses various physiological activities by specifically binding to natriuretic peptide receptor-B (abbreviated as: NPR-B, also called: GC-B) . The NPR-B includes guanylate cyclase and the CNP binding to the receptor activates its guanylate cyclase, and regulates the intracellular cGMP level. CNP acts on chondrocytes and plays important roles in bone growth (Non Patent Literature 7, Non Patent Literature 8 and so on). An elongation of bone is promoted in liver-specific CNP transgenic mice or by continuous intravenous administration of CNP-22 to normal mice (Non Patent Literature 9, Non Patent Literature 10). Also in early, basic studies, actions on expression in osteoblasts besides cartilaginous tissues, and bone resorption are reported (Non Patent Literature 11, Non Patent Literature 12). Histochemical analysis of a growth plate in a CNP transgenic mouse has revealed that 1) the growth plate thickens by elongation of the proliferating chondrocyte layer and the enlarged chondrocyte layer, 2) the extracellular matrix of the proliferating chondrocyte layer increases, and 3) the size of matured enlarged chondrocytes increases. In the enlarged chondrocyte layer located in the growth plate of the same mouse, obvious change was not observed in proliferation of chondrocytes indicated by BrdU staining, and thus it is considered that CNP promotes expression of the differentiated phenotype of chondrocytes at diverse differentiation stages of the growth plate rather than contributing to differentiation or proliferation of chondrocytes in the growth plate (Non Patent Literature 8). Further, the phenotype of an achondroplasia model mouse exhibiting the phenotype of dwarfism and contraction of long bones of the extremities was rescued by crossing with a cartilage-specific CNP transgenic mouse (Non Patent Literature 8). From this, it is supposed that CNP has the effect of ameliorating achondroplasia in the process of development or growth and various symptoms accompanying the same, and among these, the possibility of clinical application as a therapeutic drug for dwarfism represented by achondroplasia is expected (Non Patent Literature 13).

Heretofore, the usefulness of CNP for growth failure induced by the administration of a steroid has not been clear. In addition, how steroids influence the generation, secretion, and signaling pathway of CNP has been little elucidated. Currently available reports indicate that the plasma concentration of NTproCNP, which is a secretion marker of endogenous CNP, can be a biomarker of bone toxicity when dexamethasone or prednisolone is therapeutically administered to an infant or young child (aged 2 to 9) suffering from acute lymphocytic leukemia (Non Patent Literature 14), and that the number of chondrocytes and the expression level of GC-B receptor decrease while the expression level of CNP increases when dexamethasone is added to cultured mouse chondrocytes (Non Patent Literature 15). However, these reports merely indicate that the endogenous CNP or NTproCNP concentration can be an index for occurrence of growth failure caused by steroid therapy. As a bone metabolism marker that can be an index for growth failure, various substances other than the above, including bone type alkaline phosphatase, osteocalcin, type I collagen N-propeptide and type I collagen C-propeptide, are known. As is already evidenced for many molecules, variation in expression of a gene/protein in association with a physiological, pathological phenomenon does not necessarily suggest the pharmacological effect of the gene/protein, and thus it is impossible to estimate the usefulness of CNP or NPR-B receptor agonist as a medicine against growth failure induced by the administration of a steroid from the foregoing reports.

### Citation List

### Non Patent Literature

Non Patent Literature 1: Hanaoka BY et al., Rev Clin Immunol. (2012), Vol. 8, No. 8, pp. 695-697
Non Patent Literature 2 : Curtis JR et al., Arthritis Rheum. (2006), Vol. 55, No. 3, pp. 420-426
Non Patent Literature 3: Bhattacharyya A et al., Eur J Endocrinol. (2005), Vol. 153, No. 2, pp. 207-210
Non Patent Literature 4 : Allen DB et al., Endocrinol Metab Clin North Am. (1996), Vol. 25, pp. 699-717
Non Patent Literature 5: Allen DB et al., J Clin Endocrinol Metab. (1998), Vol. 83 No. 8, pp. 2824-2829
Non Patent Literature 6: Bell J et al., J Clin Endocrinol Metab. (2010), Vol. 95, No. 1, pp. 167-177
Non Patent Literature 7: Chusho H et al., Proc Nat Acad Sci. (2001), Vol. 98, No. 7, pp. 4016-4021
Non Patent Literature 8: Yasoda A et al., Nat Med. (2004), Vol. 10, No. 1, pp. 80-86
Non Patent Literature 9: Kake T et al., Am J Physiol Endocrinol Metab. (2009), Vol. 297, No. 6, pp. E1339- E1348
Non Patent Literature 10: Yasoda A et al., Endocrinology (2009), Vol. 150, No. 7, pp. 3138-3144
Non Patent Literature 11: Suda M et al., Biochem Biophys Res Commun. (1996), Vol. 223, No. 1, pp. 1-6
Non Patent Literature 12: Holiday LS et al., J Biol Chem.(1995), Vol. 270, No. 32, pp. 18983-18989
Non Patent Literature 13: Yasoda A et al., Endocr J. (2010), Vol. 57, No. 8, pp. 659-666
Non Patent Literature 14: Prickett TC et al., Peptides (2012), Vol. 36, No. 1, pp. 54-59
Non Patent Literature 15: Agoston H et al., BMC Musculoskeletal Disorders. (2006), Vol. 7, pp. 87-93

### Summary of Invention

### Technical Problem

It is an object of the present invention to provide a medicine for reducing and/or ameliorating growth failure induced by the administration of a steroid, and a medicine for treating dwarfism having occurred due to growth failure induced by the administration of a steroid.

### Solution to Problem

The present inventors have confirmed that by continuously or continually, subcutaneously administering a pharmaceutically effective dose of a steroid to a rat, growth failure appears as is reported for humans. Further, the present inventors found that growth failure caused by a steroid can be reduced and/or ameliorated by co-administering a CNP derivative to a rat in which growth failure is caused by continuous or continual subcutaneous administration of a steroid. The effect of reducing growth failure caused by a steroid by a CNP derivative was clearly strong compared with administration of the same amount of growth hormone which is an existing therapeutic drug for dwarfism. Further, it was confirmed that the action of reducing and/or ameliorating growth failure by the CNP derivative was recognized for growth failure which is induced by not only a specific steroid but also by steroids broadly. It was also confirmed that growth failure that had already occurred due to the administration of a steroid was reduced, and dwarfism caused by impairment could be treated by administration of a CNP derivative. The inventors made diligent efforts based on these findings, and finally accomplished the present invention.

The present invention provides the following invention.
(1) A medicine for reducing and/or ameliorating growth failure induced by the administration of a steroid, the medicine comprising at least one natriuretic peptide receptor B (NPR-B) agonist as an active ingredient, wherein the medicine is administered to an individual in a growth period, the individual being in need of continuous or continual administration of a steroid.
(2) The medicine according to (1), wherein the NPR-B agonist is C-type natriuretic peptide (CNP), an active fragment thereof, a mutant thereof, a derivative thereof or a modification thereof, or an anti-NPR-B antibody.
(3) The medicine according to (2), wherein the NPR-B agonist is hCNP-22 (SEQ ID NO: 1), hCNP6-22 (amino acid Nos. 6 to 22 in SEQ ID NO: 1) or hCNP-53 (SEQ ID NO: 2), a mutant thereof, a derivative thereof or a modification thereof.
(4) The medicine according to (2), wherein the NPR-B agonist is a derivative or a modification of hCNP-22 or hCNP6-22.
(5) The medicine according to any one of (2) to (4), wherein the derivative is a peptide in which at least one additional peptide selected from a peptide derived from an Fc region of immunoglobulin, serum albumin, and a partial peptide from the C-terminal end of ghrelin, is fused to one or both of the N terminus and the C terminus of hCNP-22 or hCNP6-22.
(6) The medicine according to any one of (2) to (5), wherein the derivative is a peptide having an amino acid sequence selected from SEQ ID NOs: 6 to 15.
(7) The medicine according to (6), wherein the derivative is a peptide having an amino acid sequence of SEQ ID NO: 8, 13 or 15.
(8) The medicine according to any one of (2) to (4), wherein the modification is such that PEG or a related hydrophilic polymer is conjugated to one or both of the N terminus and the C terminus of hCNP-22, hCNP6-22, or hCNP-53.
(9) The medicine according to any one of (1) to (8), wherein it is administered at the start of, or during the period of, a steroid therapy, to reduce and/or ameliorate growth failure.
(10) The medicine according to any one of (1) to (8), wherein it is administered to treat dwarfism during or after the end of a steroid therapy period, the dwarfism having occurred due to growth failure induced by the administration of the steroid.

### Advantageous Effects of Invention

An NPR-B agonist of the present invention reduces and/or ameliorates growth failure induced by the administration of a steroid, and treats dwarfism resulting from the growth failure. Further, for an infant or young child for whom the dose of steroid administered or the term over which steroids were administered was insufficient to achieve a therapeutic effect of steroids for fear of the occurrence of growth failure in conventional steroid therapy, it becomes possible to adopt a dose or a term of the steroid sufficient to achieve a therapeutic effect. It also becomes possible to improve the outcome of the primary disease by reducing and/or ameliorating growth failure induced by the administration of a steroid by combining the NPR-B agonist of the present invention with the steroid therapy. Brief Description of Drawings

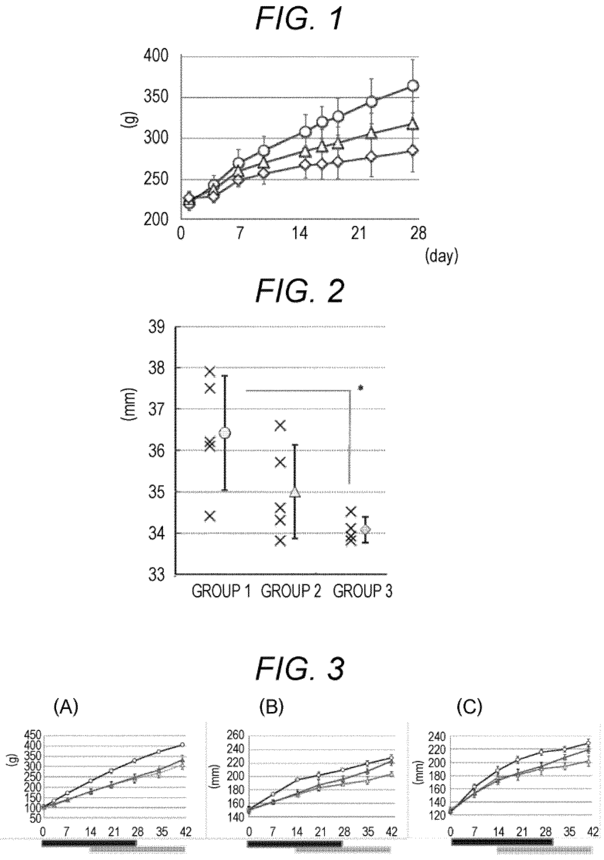

Fig. 1 is a graph showing the transition of body weight (OΔ◊ each represent mean value ± standard deviation, n = 4 to 5) when dexamethasone was subcutaneously administered to a 7-week-old male Crl: CD (SD) rat continuously for 28 days. Group 1 (○) is a normal control group, Group 2 (Δ) is a 0.5 mg/mL dexamethasone solution administration group, and Group 3 (◊) is a 1 mg/mL dexamethasone solution administration group. In Group 2 and Group 3, the solution was subcutaneously administered at a rate of 0.25 µL/hr continuously for 28 days. When there is a significant difference with the normal control group (Group 1), the data showing p < 0.05 is marked with "*", and the data showing p < 0.01 is marked with "**".

Fig. 2 is a graph showing distribution of the length of the femur after subcutaneously administering dexamethasone to a 7-week-old male Crl: CD (SD) rat continuously for 28 days ( represents individual data, ○Δ◊ represent mean value ± standard deviation). Group 1 (○) is a normal control group, Group 2 (Δ) is a 0.5 mg/mL dexamethasone solution administration group, and Group 3 (◊) is a 1 mg/mL dexamethasone solution administration group. In Group 2 and Group 3, the solution was subcutaneously administered at a rate of 0.25 µL/hr continuously for 28 days. The data marked with "*" showed a significant difference (p <0.05) with the normal control group.

Fig. 3 is a graph showing transitions in the body weight (Fig. 3A), the body length (Fig. 3B) and the tail length (Fig. 3C) (each data is mean value ± standard deviation, n = 5) when a 0.67 mg/mL dexamethasone solution was subcutaneously administered to a 4-week-old male Crl : WI (Wistar) rat at a rate of 0.25 µL/hr continuously for 28 days (the period of the black bar below the graph). At 14 days after starting the administration, when the obvious growth failure due to dexamethasone was confirmed, continuous subcutaneous administration in combination with CNP derivative (A) was started, and the administration was continued for 28 days (the period of the gray bar below the graph). Group 1 (○) is a normal control group, Group 2 (Δ) is a single administration group of dexamethasone, Group 3 (▲) is a combined administration group of dexamethasone and CNP derivative (A). Administration of dexamethasone was continued for 28 days, and a 15 mg/mL CNP derivative (A) solution was subcutaneously administered at a rate of 0.25 µL/hr continuously for 28 days from 14 days after starting the administration of dexamethasone.

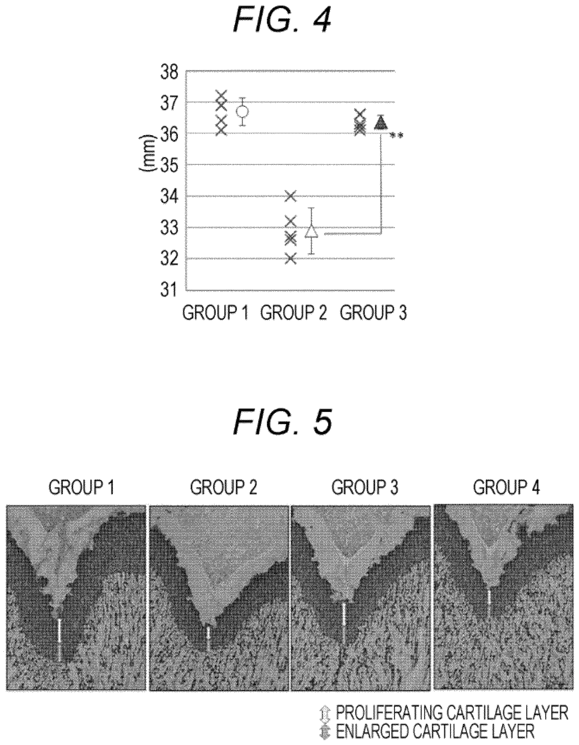

Fig. 4 shows the distribution of the length of the femur at the end of a test when a 0.67 mg/mL dexamethasone solution was subcutaneously administered to a 4-week-old male Crl: WI (Wistar) rat at a rate of 0.25 µL/hr continuously for 28 days, and at 14 days after starting the administration, when the obvious growth failure due to dexamethasone was confirmed, continuous subcutaneous administration in combination with CNP derivative (A) was started (× represents individual data, ◊Δ▲ represent mean value ± standard deviation). Group 1 (○) is a normal control group, Group 2 (Δ) is a single administration group of dexamethasone, Group 3 (▲) is a combined administration group of dexamethasone and CNP derivative (A). Administration of dexamethasone was continued for 28 days, and a 15 mg/mL CNP derivative (A) solution was subcutaneously administered at a rate of 0.25 µL/hr continuously for 28 days from 14 days after starting administration of dexamethasone. The data marked with "**" showed a significant difference (p < 0.01) with the normal control group (Group 2).

Fig. 5 illustrates histological images of growth plates (toluidine blue stained) of the femur epiphyseal region at the end of a test in which a 0.67 mg/mL dexamethasone solution was subcutaneously administered to a 4-week-old male Crl: WI (Wistar) rat at a rate of 0.25 µL/hr continuously for 28 days, and CNP derivative (A) for Group 3 or human-type growth hormone for Group 4 were subcutaneously administered in combination with the dexamethasone solution once a day repeatedly for 28 days. The normal group for which administration was not made was named Group 1, and the single administration group of dexamethasone was named Group 2. In the histological images, a proliferating cartilage layer is indicated by the white double-headed arrow, and an enlarged cartilage layer is indicated by the gray double-headed arrow.

### Description of Embodiments

Hereinafter, the present invention will be specifically described.

The present invention relates to a medicine for reducing and/or ameliorating growth failure induced by the administration of a steroid, and a medicine for treating dwarfism having occurred due to growth failure induced by the administration of a steroid, the medicine containing at least one natriuretic peptide receptor B (NPR-B) agonist as an active ingredient.

### <NPR-B agonist>

In the present invention, a "natriuretic peptide receptor B agonist" means a substance binding to an NPR-B (Natriuretic Peptide Receptor-B, also known as: Guanylate Cyclase B (GC-B), sometimes referred to as "NPR-B"), and having the effect of activating the guanylate cyclase thereof (hereinafter, "NPR-B agonist activity"), and is sometimes referred to simply as a "NPR-B agonist" in the present specification. Examples of a representative NPR-B agonist include, for example, C-type natriuretic peptide (CNP). The NPR-B agonist of the present invention is not particularly limited as long as the NPR-B agonist is a substance having NPR-B agonist activity; and CNP, and an active fragment thereof; a mutant thereof; a derivative thereof; a modification thereof; and the like can be used. Further, the agonist of the present invention includes a peptide or a small molecule compound that does not have structural similarity with CNP, as long as such substance has NPR-B agonist activity.

Examples of the CNP in the present invention include human-derived CNP-22 consisting of 22 amino acids (hCNP-22: GLSKGCFGLK LDRIGSMSGL GC: SEQ ID NO: 1, having a common amino acid sequence in mammals such as pigs and rats), human-derived CNP-53 (hCNP-53, amino acid sequence: DLRVDTKSRA AWARLLQEHP NARKYKGANK KGLSKGCFGL KLDRIGSMSG LGC: SEQ ID NO: 2), chicken-derived CNP-22 (GLSRSCFGVK LDRIGSMSGL GC: SEQ ID NO: 3), and frog-derived CNP-22 (GYSRGCFGVK LDRIGAFSGL GC: SEQ ID NO: 4).

It is considered that in the various kinds of CNP, a ring structure formed by a disulfide bond between two C residues contained in the sequence (for example, in hCNP-22, a disulfide bond is formed between C at position 6 and C at position 22 in SEQ ID NO: 1, and a ring structure is formed) is important for binding to the NPR-B receptor and for activity (Furuya, M. et al, Biochem. Biophys. Res. Commun., (1992), Vol. 183, No. 3, pp. 964-969; Silver, MA, Curr. Opin. Nephrol. Hypertens., (2006), Vol. 15, pp. 14-21; and Calderone, A., Minerva Endocrinol., (2004),Vol. 29, pp. 113-127). According to the report of Furuya et al. , it has been reported that CNP6-22, which is a peptide consisting of only the ring structure (a peptide consisting of amino acid Nos. 6 to 22 of SEQ ID NO: 1), or even a peptide in which the sequence on the N-terminal side and the sequence on the C-terminal side of the ring structure of ANP is added to the N terminus and the C terminus of the ring structure, respectively, show almost the same NPR-B agonist activity as that of hCNP-22. The report also shows that a peptide having mutations in L at position 9 and K at position 10 of hCNP-22, attenuates the activity, but that a peptide having a mutation at a position other than the above (for example, S at position 16 and M at position 17), and a peptide in which the amino acid sequence of positions 10 to 12 of ANP is substituted for L-K-L, which is the corresponding sequence of hCNP-22 (positions 9 to 11 of SEQ ID NO: 1), show almost the same NPR-B agonist activity as that of hCNP-22; and the like. From these findings, the amino acid sequence of the ring structure that is important for NPR-B agonist activity is CFGLKLDRIG-Xaa1-Xaa2-SGLGC (herein, Xaa1 represents S or A, and Xaa2 represents M, F or E; SEQ ID NO: 5), and the sequence is referred to as a "ring structure sequence".

In the present invention, the "active fragment" of a peptide or protein having biological activity means an active fragment that is composed of a region related to biological activity of the peptide or protein, and that retains at least part of the biological activity that is possessed by the peptide or protein. As an active fragment of CNP in the present invention, a peptide that consists of at least part of the amino acid sequence of any one of the amino acid sequences of SEQ ID NOs: 1 to 4, and has NPR-B agonist activity can be used. Examples of an active fragment include a peptide consisting of the above-described ring structure sequence (the amino acid sequence of SEQ ID NO: 5), and specific examples include hCNP6-22; a peptide of SEQ ID NO: 1, 3 or 4 in which some or all of the amino acids at positions 1 to 5 of the amino acid sequence are deleted wherein the deletion includes consecutive amino acids containing position 1; and a peptide of SEQ ID NO: 2 in which some or all of the amino acids at positions 1 to 31 of the amino acid sequence are deleted wherein the deletion includes consecutive amino acids containing position 1. However, the active fragment is not limited thereto, and any active fragment having a ring structure sequence and NPR-B agonist activity can be employed as the active fragment of CNP of the present invention.

As an NPR-B agonist of the present invention, the above-described active fragment itself can be employed, and further, a peptide (a derivative of an active fragment) in which one or more amino acids are added to the N terminus, the C terminus, or both thereof, of the active fragment, can also be employed as long as it retains the intended agonist activity. Examples of such a peptide can include a peptide in which a sequence derived from the C terminus and N terminus of an ANP is added to the N terminus, C terminus, or both thereof, of hCNP6-22 (Furuya, M. et al., Biochem. Biophys. Res. Commun., (1992), Vol. 183, No. 3, pp. 964-969).

In the present invention, the "mutant" of a peptide or protein having biological activity means a mutant in which one to several amino acids are substituted, deleted, inserted, and/or added (hereinafter, referred to as "substitution and the like") in one to several regions in an amino acid sequence of the peptide or protein, and that retains at least part of the biological activity that is possessed by the peptide or protein. The term "several regions" means usually 3 regions, preferably 2 regions. The term "several amino acids" means usually 10 amino acids, preferably 5 amino acids, more preferably 3 amino acids, and even more preferably 2 amino acids. In cases where substitution and the like are performed at multiple regions, only any one of substitution, deletion, insertion, and addition may be performed, or the substitution, deletion, insertion, and addition may be performed in a combination of two or more thereof. Further, the amino acid used for substitution and the like may be a naturally-occurring amino acid, may be a modification such as an acylated form of a naturally-occurring amino acid, or may be an artificially synthesized amino acid analog. In addition, any of the regions in which substitution and the like are performed may be selected as long as part of the activity of the original peptide or protein is retained, however, it is preferable that the region at which substitution and the like are performed is a region other than the active region or receptor binding region of the original peptide or protein.

For example, as the mutant of CNP, a mutant in which substitution and the like have been performed at any region can be employed as long as the NPR-B agonist activity is retained, however, the mutant of CNP preferably includes a peptide in which the above-described ring structure sequence is retained, and the substitution and the like have been performed in a region other than the region of the ring structure sequence. The specific mutant of CNP may be a mutant in which substitution and the like of one to several amino acids may be performed in the intended one or more regions in the amino acid sequence described in SEQ ID NOs: 1 to 4 as long as the NPR-B agonist activity is possessed, however, it may preferably be a mutant in which substitution and the like of one to several amino acids are performed in one to several regions at an amino acid other than an amino acid shown in SEQ ID NO: 5 in an amino acid sequence described in any one of SEQ ID NOs : 1 to 4, and may more preferably be a mutant in which substitution and the like of one to several amino acids are performed in one to several regions at positions 1 to 5 of an amino acid sequence described in SEQ ID NO: 1, 3 or 4, or a mutant in which substitution and the like of one to several amino acids are performed in one to several regions at positions 1 to 31 of the amino acid sequence described in SEQ ID NO: 2.

As the NPR-B agonist of the present invention, the above-described mutant itself can be employed, and further, a peptide (a derivative of a mutant) in which one or more amino acids are added to the N terminus, the C terminus, or both thereof of the mutant, can also be employed, as long as the intended agonist activity is retained.

As specific examples of the mutant of CNP, there are reports that a peptide with mutations at position 17 and position 18 of hCNP-22 has the same NPR-B agonist activity as that of hCNP-22; that even in a derivative of a mutant in which the N terminus and C terminus of a ring structure element of such a mutant are replaced with sequences derived from ANP, it has been shown that the NPR-B agonist activity is around 90% of the activity of hCNP-22; that a peptide with a mutation at any of positions 9 to 11 shows NPR-B agonist activity that is 50% or more of hCNP-22; that a peptide with mutations at both of positions 10 and 11 has NPR-B agonist activity that is 40% or more of hCNP-22; and the like (Furuya, M. et al., Biochem. Biophys. Res. Commun., (1992), Vol. 183, No. 3, pp. 964-969). In addition, in another literature reference, it has been described that various kinds of mutants of hCNP-22 retain NPR-B agonist activity; and further have resistance to cleavage by neutral endopeptidase (NEP) which is a catabolic enzyme of CNP (WO 2009/067639 pamphlet).

In the present invention, the "derivative" of a peptide or protein having biological activity means a fusion peptide containing an amino acid sequence of the peptide or protein to which, further, another peptide or protein is added, and at least part of the biological activity that is possessed by the original bioactive peptide or protein is retained. A fusion peptide having at least part of such biological activity (in the present invention, the effect of binding to NPR-B and activating the guanylate cyclase) is also referred to as a derivative of a bioactive peptide. In the derivative of the present invention, an additional peptide may be fused at one of the C terminus or the N terminus of the original bioactive peptide or protein, or additional peptides may be fused at both the C terminus and the N terminus of the original bioactive peptide or protein. As the peptide to be added, the peptide is not particularly limited, however, a peptide that does not have its own bioactivity is preferable. In addition, the additional peptide may be bonded directly, or may be bonded via a linker sequence consisting of one to several amino acids. As the linker sequence, various linker sequences are known, however, a linker sequence containing many G, S, and the like is frequently used. Examples of such an additional peptide include an Fc region of immunoglobulin (preferably, IgG), serum albumin, and a partial sequence from the C-terminal side of ghrelin. Examples of the derivative used as the NPR-B agonist of the present invention can include a derivative of CNP (preferably, hCNP-22 or hCNP-53), and a derivative of an active fragment of CNP (preferably, hCNP6-22), and is preferably a derivative of hCNP-22, or a derivative of hCNP6-22.

Specific examples of the derivative of CNP can include, for example, various kinds of CNP derivatives disclosed in the publication of US patent application US 2010-305031 (corresponding to International Publication WO2009/142C307) (SEQ ID NOs: 109, 110, 124 to 130, 157 and 158 in the publication of the US patent application: SEQ ID NOs: 6 to 14 in the present specification). Therein, it has been reported that in a derivative in which a partial sequence derived from the C-terminal side of ghrelin is added to a bioactive peptide such as ANP, CNP, and motilin, the half-life time in blood is improved while the bioactivity of the original peptide is retained. In that report, in any of the various derivatives in which a peptide containing Wk-Xl-Y-Zm-Wn derived from the C terminus of ghrelin (herein, W represents a basic amino acid such as Lys, and Arg; Y represents an acidic amino acid such as Asp, and Glu; X and Z are the same as or different from each other, and may be any amino acid except for acidic amino acids and basic amino acids; k and n are independent from each other, and are an integer of 1 or 2; 1 and m are independent from each other, and are a natural number of 0, 1 or 2; and examples of such a sequence preferably include RKESKK, RKDSKK, RKSEKK, and RKSDKK) was added to either one of the N terminus or the C terminus of CNP or both thereof, the NPR-B agonist activity was also retained, and the half-life in blood was prolonged. Any of CNP derivative (A) (SEQ ID NO: 13) and CNP derivative (C) (SEQ ID NO: 8) used in the Examples of the present invention are representative examples of CNP derivatives prepared in this publication. The description of this publication is incorporated within the description of the present specification.

Further, as regards CNP or a derivative of the active fragment of CNP, NPR-B agonist activity of around the same level as that of hCNP-22 was retained even in a peptide in which the C-terminal part of ANP was added to the C terminus of hCNP-22, or a peptide in which the N-terminal part and the C-terminal part of an ANP were added to the N terminus and the C terminus of hCNP6-22 (a derivative of CNP active fragment) (Furuya, M. et al., Biochem. Biophys. Res. Commun., (1992), Vol. 183, No. 3, pp. 964-969). In addition, in another literature reference, it has been described that various kinds of derivatives of hCNP-22 and hCNP-53 retain NPR-B agonist activity; further, multiple derivatives among the various kinds of derivatives have NEP degradation resistance; and the like (WO 2009/067639 pamphlet). Further, for the derivative described in this WO 2009/067639 pamphlet, a pharmacological effect on a mouse achondroplasia model of a derivative composed of an amino acid sequence of Pro-Gly-CNP-37 (SEQ ID NO: 15), which is defined as CNP derivative (B) in the present specification, has also been reported (Florence L., et al., Am. J. Hum. Genet., (2012), Vol. 91, No. 6, pp 1108-1114: body text:
http://www.sciencedirect.com/science/article/pii/S000292971 200537X: Supplement::
http://download.cell.com/AJHG/mmcs/journals/0002-9297/PIIS0 00292971200537X.mmcl.pdf).

Examples of the derivative of CNP-22 of the present invention include a peptide consisting of an amino acid sequence in which 1 or more to 30 or fewer amino acids consecutive from the N terminus are deleted from the amino acid sequence (SEQ ID NO: 2) of human CNP-53, specifically, CNP-36 (amino acid Nos. 18 to 53 of SEQ ID NO: 2). Such a derivative is preferably a peptide consisting of an amino acid sequence in which 10, 11, 12, 13, 14, 15, 16, 17, 18, 19, 20, 21, 22, 23, 24 or 25 amino acids consecutive from the N terminus of hCNP-53 (SEQ ID NO: 2) are deleted, and more preferably, hCNP-36 in which 17 consecutive amino acids from the N terminus of hCNP-53 are deleted (a peptide consisting of the amino acid sequence of amino acid Nos. 18 to 53 of SEQ ID NO: 2).

These various CNP derivatives and a derivative of CNP active fragment can preferably be used as the NPR-B agonist of the present invention. The NPR-B agonist of the present invention is more preferably a derivative consisting of an amino acid sequence in which 1 or more to 30 or fewer consecutive amino acids from the N terminus (preferably, 25 or fewer, and more preferably 20 or fewer) are deleted from SEQ ID NO: 2, or any derivative selected from SEQ ID NOs : 6 to 15; and furthermore preferably CNP derivative (A) (SEQ ID NO: 13), CNP derivative (C) (SEQ ID NO: 8), or CNP derivative (B) (SEQ ID NO: 15).

In the present invention, the "modification" of a peptide or protein having biological activity means a modification in which one to several regions of amino acids contained in the peptide or protein are modified by chemical reaction with another chemical substance, and further, at least part of the biological activity that is possessed by the peptide or protein is retained after the modification. As the region in which the modification is performed, any region may be selected as long as the activity of the original peptide or protein is retained in the modification.

For example, for a modification in which the chemical substance is large to some extent, such as a polymer, the modification is preferably performed at a region other than an active region or the receptor binding site of the peptide or protein. Further, in the case of a modification for preventing cleavage by a catabolic enzyme, modification performed at the cleavage site is preferable.

For example, as long as NPR-B agonist activity is possessed, the modification of CNP may be a modification in which the intended one or more regions in the amino acid sequence of any one of SEQ ID NOs : 1 to 4 are modified. However, preferably a modification in which one to several regions, at an amino acid other than in the amino acid sequence shown in SEQ ID NO: 5, in the amino acid sequence of any one of SEQ ID NOs: 1 to 4 are modified, and more preferably a modification in which one to several regions at positions 1 to 5 of the amino acid sequence in SEQ ID NO: 1, 3 or 4 are modified. Further, in the case of a modification conferring resistance against NEP cleavage, it is known that NEP cleaves CNP at the site between C at position 1 and F at position 2 of SEQ ID NO: 5 in the ring structure, which is contained in various kinds of CNP peptides and, therefore, this region can be modified.

Furthermore, a modification of the above-described CNP active fragments, CNP mutants and derivatives thereof is also included in the present invention. Such various kinds of modifications can also be used in the present invention as long they retain NPR-B agonist activity.

As a method of chemical modification, various methods are known, for example, a method of adding a high-molecular weight polymer that is used in pharmaceutical techniques (pharmacologically used), such as polyethylene glycol (PEG), and polyvinyl alcohol (PVA); a method in which a compound that is to be a linker is added to a side chain of an amino group such as a K residue, via which another protein or the like (for example, serum albumin) are bonded; and the like have been known. However, the method is not limited thereto, and various methods can be employed.

As a specific example of such modification of CNP, the active fragment thereof, the mutant thereof and the derivative thereof, those disclosed in the WO 2009/067639 pamphlet are exemplified. There is a disclosure in the reference that a modification in which various kinds of aqueous polymers such as PEG are bonded to hCNP-22 and hCNP-53, and multiple modifications in which the peptide bond of Cys6-Phe7, which is a cleavage site for NEP in hCNP-22, is substituted with a pseudo-peptide bond of -CH₂-NH-, -C(=O)-N(R)- (wherein R represents a lower alkyl group such as a methyl group, an ethyl group, a propyl group, an isopropyl group, an n-butyl group, an isobutyl group, a sec-butyl group, or a tert-butyl group), or the like retain NPR-B agonist activity, and many of which have improved half-life time in blood compared with that of hCNP-22. Further, as for the production method of a modification of various bioactive peptides, the modification can, for example, appropriately be prepared with reference to the publication of US patent application US 2009-0175821 and the like.

For the binding of CNP to an NPR-B receptor, the ring structure thereof is important. Therefore, particularly in a derivative or modification in which an additional peptide or moiety is bonded to the terminal part thereof, such an additional peptide or moiety affects the ring structure less, and the derivative or modification can be expected to retain NPR-B agonist activity to a sufficient degree without inhibiting binding to the NPR-B receptor. This is supported by many of the literature references described above.

The above-described CNP, or the active fragment thereof; or the mutant thereof, the derivative thereof, or the modification thereof may be that which is collected from a natural cell or tissue, may be that which is produced by using a genetic engineering or cell engineering technique, maybe that which is synthesized chemically, or may be that in which those obtained as above are enzymatically treated or chemically treated to modify the amino acid residue or to remove part of the amino acid sequence. These substances can appropriately be produced in accordance with conventional methods with reference to the description of the present specification, and the cited literature.

It can easily be measured, by a conventional method known by a person skilled in the art, whether a certain substance has NPR-B agonist activity or not. Specifically, the method is that a substance is added to a cultured cell in which NPR-B (Suga S., et al., Endocrinology, (1992), Vol. 130, No. 1, pp. 229-239) is forcibly expressed, and the intracellular cGMP level of the cultured cell is measured. The phrase "part of the NPR-B agonist activity is retained" means that when the NPR-B agonist activity is measured for an NPR-B agonist substance and CNP on the same test, the peak of cGMP increasing activity of the NPR-B agonist substance retains at least 10% or more of that of CNP, and preferably retains 30% or more, more preferably 50% or more, and even more preferably 70% or more. Further, even if the activity of the NPR-B agonist substance is not significantly increased at its peak value, one that shows favorable results as regards activity duration and half-life time in blood when administered to a living body, can be used in the present invention.

In addition, a compound, even one which does not have a structure in common with a natriuretic peptide (for example, a small molecule compound), can be used in the present invention as an NPR-B agonist, as long as it is a compound which, when added to the above-described test method, results in the cGMP production ability being improved.

Further, it is in accordance with the ordinary knowledge in the art to select, using a method described above, an antibody having NPR-B agonist activity from among the anti-NPR-B antibodies prepared by immunizing an animal with an antigenic protein containing a ligand binding site of NPR-B. The thus-prepared anti-NPR-B antibody having NPR-B agonist activity can also be used as the NPR-B agonist of the present invention. In addition, the thus-used antibody can be prepared in various known forms, that is, an antibody produced by an immunogenic animal, a chimeric antibody, a CDR-grafted antibody, a humanized antibody, a fully human antibody, and the like. An antibody fragment such as a Fab, and an scFv, which are prepared based on the above-described antibodies, can be used.

Examples of the NPR-B agonist of the present invention preferably include CNP, an active fragment having the ring structure sequence thereof, or a mutant in which substitution and the like are performed in a sequence other than the ring structure sequence, or a derivative or modification thereof; more preferably include hCNP-22, hCNP-53, hCNP6-22, a derivative thereof or a modification thereof; and even more preferably include hCNP-53, CNP derivative (A): CNP (1-22) Ghrelin (12-28, E17D, SEQ ID NO: 13), CNP derivative (B): Pro-Gly-CNP-37 (SEQ ID NO: 15), CNP derivative (C) : CNP (1-22) Ghrelin (12-28, SEQ ID NO: 8) and partial peptides thereof.

### <Administration subject>

The NPR-B agonist of the present invention can be used as an active ingredient of a medicine for reducing and/or ameliorating growth failure induced by the administration of a steroid to an individual in a growth period, which individual is in need of continuous or continual administration of a steroid, by being administered to such a subject.

In the present invention, "an individual in a growth period, which individual is in need of continuous or continual administration of a steroid" means a subject in a growth period suffering from a disease that is curable by a steroid, for which administration of a steroid is scheduled, or is being conducted, or has been completed in a dosage and/or for an administration period that can suppress or inhibit bone elongation during normal growth. The subject to which the medicine of the present invention is administered is not particularly limited as long as it is a mammal, and examples include a human, monkey, dog, cat, and horse, with a human being preferred. In the present invention, "growth period" means a period during which the growth plate is kept in a condition such that the epiphyseal line of a long bone of the subject is not closed, and in the case of a human, it is typically 18 years or younger, preferably 15 years or younger, more preferably 12 years or younger. Since the medicine of the present invention reduces and/or ameliorates growth failure, the effect is more likely to be significant by the medicine being administered to a subject in a period in which the growth speed is high.

In the present invention, a "steroid" is an agent containing a glucocorticoid or a compound related thereto as an active ingredient, used for a therapeutic purpose in relation to a disease in medical situations. Representative examples include agents containing prednisolone, beclometasone, betamethasone, fluticasone, dexamethasone, hydrocortisone or the like as an active ingredient (Payne et al., Paediatr Respir Rev., (2001), Vol. 2, pp. 145-150, Pelaia G et al., Life Sci., (2003), Vol. 72, No. 14, pp. 1549-1561), however, it is not limited thereto. The preparation form of the steroid of the present invention is not limited, and may be an oral preparation, an injection, an external preparation and so on.

In the present invention, "growth failure induced by the administration of a steroid" means the entirety of the generation and progression of events as will be described below and the appearance resulting from said generation and progression. Long-term use of a steroid in a subject in a growth period is associated with the risk of the occurrence of growth failure. Long-term therapy with a steroid in a growth period results in the prevention of the generation and secretion of growth hormone and insulin-like growth factor (IGF-1) by glucocorticoids that are excessive in the body, or results in deterioration in sensitivity to such hormones. This causes a condition in which normal growth is suppressed, namely "growth failure", and can result in the diagnosis of so-called "dwarfism" which is a condition in which height is significantly lower in comparison with the standard growth curve of subjects of the same age (Kawaguchi H et al., J Bone Miner Res. (2001), Vol. 25, No. 12, pp. 2735-2743).

Examples of the disease for which therapy with a steroid for a relatively long term is required in a subject in a growth period, namely a disease from which the subject to be administered with the medicine of the present invention is suffering, or suffered in the past, include asthma, atopic dermatitis, systemic lupus erythematosus, rheumatic fever, juvenile idiopathic arthritis, ulcerative colitis, Crohn's disease, myasthenia gravis, Guillain-Barre syndrome, pulmonary hemosiderosis, infantile IgA nephropathia, idiopathic thrombocytopenic purpura, autoimmune hemolytic anemia, aplastic anemia, acute lymphocytic leukemia, and congenital adrenal hyperplasia. At least some of these are such serious diseases that it is difficult to stop therapy with a steroid, and death can be caused if the diseases become worse. Therefore, there is a demand to minimize the appearance of various side effects while continuing to use a steroid within the range that attains the intended effect to a sufficient degree.

In the present invention, "reduce" a growth failure means following such a course that by achieving at least one of the effects of suppressing occurrence of impairment, alleviating or delaying the progression of impairment, and reducing the degree of impairment, dwarfism that can occur as a result of growth failure does not occur, or alleviation of the degree of dwarfism is expected. For the purpose of reducing growth failure, it is desired that administration of the medicine of the present invention is started directly before or simultaneously with the start of steroid therapy, or during steroid therapy, and it is preferred that administration is continuously or continually conducted until the end of the steroid therapy or following a lapse of a certain period thereoafter

In the present invention, "ameliorate" a growth failure means bringing the height of a subject to a standard height range (of an individual) of the same age by achieving at least one of the following effects: improving the growth rate in the subject to be comparable with, or better than, the standard growth rate of the same age; preventing the occurrence of dwarfism in the course of progression of growth failure; recovering dwarfism that has occurred; and, after end of the event inducing the impairment, recovering in such a manner that the resultant impaired condition is brought closer to the normal condition. For the purpose of ameliorating growth failure, administration of the medicine of the present invention is started for a subject in a growth period who has undergone a certain period of steroid therapy or completed the therapy, and exhibits a tendency towards dwarfism, or suffers from dwarfism. When administration is performed during the period of steroid therapy, both the reducing effect and the ameliorating effect are expected to occur.

The medicine of the present invention is not particularly limited as long as it is a medicine containing the NPR-B agonist of the present invention as an active ingredient, that it is formulated for a subject in a growth period in need of continuous or continual administration of a steroid, for the purpose of reducing and/or ameliorating growth failure induced by the administration of a steroid in the subject, or for treating dwarfism having occurred by growth failure. However, the package insert of the medicine preferably includes a description to the effect that the medicine can be administered to a subject undergoing steroid therapy, the medicine is administered to a subject in danger of the occurrence of growth failure due to the administration of a steroid, or a subject to whom growth failure has occurred, and the medicine of the present invention is administered for the purpose of reducing and/or ameliorating the growth failure, or for treating dwarfism having occurred as a result of the growth failure.

### <Form of medicine>

The medicine of the present invention is capable of reducing and/or ameliorating growth failure induced by the administration of a steroid or treating dwarfism having occurred due to growth failure when administration of the medicine is started directly before or simultaneously with the start of a steroid therapy or during a steroid therapy in the administration subject, and the medicine is continually administered during the period of the steroid therapy. In this case, in the present invention, the phrase "the medicine of the present invention is administered continuously or continually during the period of the steroid therapy" means that the subject to whom the medicine is to be administered receives all of the active ingredients (steroid and NPR-B agonist of the present invention) into his/her body for a certain period. A preparation containing all of the active ingredients in a single preparation may be administered, or each active ingredient may be separately formulated, and each active ingredient may be administered separately. When they are formulated separately, the administration time is not particularly limited, and they may be administered simultaneously, or may be administered at different times at a certain interval, or on different days. The expression that a plurality of active ingredients or drugs are "administered in combination", also includes the case where a plurality of active substances acting as an NPR-B agonist are singly administered. When a plurality of active ingredients are administered at different times or on different days, the order of administration of the active ingredients is not particularly limited. Since different formulations are typically administered according to their respective administration methods, they can be administered the same number of times or a different number of times. Also when the active ingredients are formulated separately, the respective administration methods (administration routes) of the formulations may be the same, or they may be administered by different administration methods (administration routes). Further, it is not necessary that all of the active ingredients are present simultaneously in the body, but it is only required that each active ingredient is taken into the body in a certain period (for example, one month, preferably one week, more preferably several days, further preferably one day), and one active ingredient may have disappeared from inside the body when another active ingredient is administered.

The medicine of the present invention is capable of treating dwarfism having occurred due to growth failure induced by the administration of a steroid, by being administered during or after the end of a steroid therapy. As for the term "dwarfism", generally, a height of less than or equal to a standard height minus -2 SD, in comparison with the height of the same race, the same sex and the same age, or a growth speed in two years of less than or equal to -1.5 SD is defined as dwarfism. Administration in this case is conducted for a subject during a growth period as long as the standard height +2 SD at the age of the subject is not exceeded. Even in a case not agreeing with the definition of dwarfism, a patient in a growth period undergoing continuous or continual steroid therapy or having a history of such therapy, who has obviously low height compared with the standard height of the same race, the same sex and the same age, or who exhibits significantly decreased growth speed, is suspected to suffer from growth failure, and hence the patient can also be a subject for administration of the medicine of the present invention.

Further, by the action of the medicine of the present invention as described above, it becomes possible to increase the amount of a steroid used for the therapy of the primary disease and to prolong the therapeutic period as a result of reduction of the risk of growth failure due to the administration of a steroid, so that the outcome of the primary disease can be improved, and the present invention also provides a drug having such an action.

The substance that is used as the active ingredient of the medicine of the present invention may be a pharmaceutically acceptable salt of the above-described NPR-B agonist. That is, in the present invention, an acid addition salt of an inorganic acid, for example, hydrochloric acid, sulfuric acid, or phosphoric acid, or an organic acid, for example, formic acid, acetic acid, butyric acid, succinic acid, citric acid, or the like, of the above-described NPR-B agonist can also be used as an active ingredient. Alternatively, in the present invention, the form of a metal salt of sodium, potassium, lithium, calcium, or the like, or a salt with an organic base, of the above-described NPR-B agonist can also be used as the active ingredient. Further, the pharmaceutical composition of the present invention may comprise a free form of a substance in accordance with the active ingredient, or a pharmaceutically acceptable salt thereof.

The pharmaceutical composition of the present invention contains an NPR-B agonist, or a pharmaceutically acceptable salt thereof as an active ingredient, and further is prepared by using a carrier, an excipient, other additives, a diluent, or the like that are used during ordinary formulation. Examples of the carrier or excipient for the formulation include, for example, lactose, magnesium stearate, starch, talc, gelatin, agar, pectin, gum arabic, olive oil, sesame oil, cacao butter, ethylene glycol, and other ones that are usually used.

As the solid composition for oral administration, tablets, pills, capsules, powders, granules, and the like are used. In such a solid composition, at least one active ingredient is mixed with at least one inactive diluent, for example, lactose, mannitol, glucose, hydroxypropyl cellulose, microcrystalline cellulose, starch, polyvinylpyrrolidone, magnesium aluminometasilicate, and the like. The composition may contain an additive other than the inactive diluent, for example, a lubricant such as magnesium stearate, a disintegrating agent such as cellulose calcium glycolate, and a solubilizer such as glutamic acid or aspartic acid in accordance with conventional methods. The tablet or pill may be coated as needed with a sugar coat such as sucrose, gelatin, or hydroxypropylmethylcellulose phthalate, or a film made from a gastro- or enteric-soluble substance, or may be coated with two or more layers. In addition, a capsule made from a substance such as gelatin, which can be absorbed, is also included.

A liquid composition for oral administration contains a pharmaceutically acceptable emulsifier, a solution agent, a suspending agent, a syrup, an elixir or the like, and may further contain an inactive diluent that is usually used, for example, purified water, ethanol, or the like. This composition may contain an adjuvant such as a wetting agent, and a suspending agent other than the inactive diluent; a sweetening agent; a flavor; an aromatic; a preservative; or the like.

As the injectable formulation for parenteral administration, a solution agent, a suspending agent, and an emulsifier are included under sterile and aqueous form or non-aqueous form. As the aqueous solution agent or suspending agent, an injection solvent, and an injectable saline solution are, for example, included. As the non-aqueous solution agent, and suspending agent, propylene glycol, polyethylene glycol, vegetable oil such as olive oil, alcohols such as ethanol, Polysorbate 80 (registered trademark), and the like are, for example, included. Such a composition may further contain an adjuvant such as a preservative, a wetting agent, an emulsifier, a dispersant, a stabilizing agent (for example, lactose), and a solubilizer (for example, glutamic acid and aspartic acid). These can be sterilized, for example, by filtering sterilization with a microfiltration membrane, heat sterilization such as a high pressure steam sterilization, or an ordinary sterilization method of mixing a germicide, or the like. The injection may be a solution formulation, or may be a lyophilized formulation that is dissolved and reconstituted before use. As the excipient for the lyophilization, for

### example, a sugar alcohol such as mannitol, or glucose, or saccharides can be used.

The pharmaceutical composition of the present invention is preferably administered by an administration method commonly used for pharmaceuticals, for example, an oral administration method; or a parenteral administration method of transmucosal administration, intravenous administration, intramuscular administration, subcutaneous administration, or the like. In cases where the active ingredient is an NPR-B agonist antibody, the pharmaceutical composition of the present invention is usually administered via a parenteral route, for example, via injection (subcutaneous injection, intravenous injection, intramuscular injection, intraperitoneal injection, etc.), via the skin, via the mucous membrane, via the nose, via the lungs, or the like, however, it can also be administered by oral administration.

When the active ingredient is a peptidic substance, administration may be conducted by oral administration as a pharmaceutical formulation that is less susceptible to degradation in the digestive tract, for example, as a micro capsule formulation in which a peptide which is an active ingredient is enclosed in a ribosome. Further, an administration method in which the active ingredient is absorbed via the mucous membrane of the rectum, nose, hypoglottis, or the like, other than the digestive tract, can also be used. In this case, administration to an individual can be conducted in the form of a suppository, a nasal spray, an inhalant, or a sublingual tablet.

The dosage of a substance that can be used as an active ingredient of the pharmaceutical composition according to the present invention varies depending on the type of primary disease; the age, and the body weight of the individual (subject) ; the degree of symptoms; and the administration route, however, for a human, it is, for example, around 100 mg/kg or less, preferably around 50 mg/kg or less, and more preferably 5 mg/kg or less, generally as the upper limit of the dosage per day. Further, as the lower limit of the dosage per day, the dosage is, for example, around 0.005 µg/kg or more, preferably 0.025 µg/kg or more, and more preferably 0.1 µg/kg or more.

The dosage of CNP in the present invention varies depending on the body weight of the subject individual, the disease to be treated, the symptoms, and the like, however, it is around the level of 10⁻¹⁵ M to 10⁻⁶ M, and preferably around the level of 10⁻¹² M to 10⁻⁸ M at the tissue level in the topical administration. For systemic administration, the dosage is around the level of 0.01 µg/head to 10000 µg/head, and preferably 1 µg/head to 5000 µg/head.

The pharmaceutical composition of the present invention is used in combination with a steroid, or administered to the subject that has been administered with a steroid in the past. The pharmaceutical composition of the present invention may be used in combination with another bone formation promoter, bone resorption inhibitor, or dwarfism therapeutic agent. Examples of the agent to be combined include, for example, a bisphosphonate agent, a bone morphogenetic protein (BMP) agent, a parathyroid hormone (PTH) agent, a basic fibroblast growth factor (bFGF) agent, an anti-osteoclast differentiation factor (RANKL) antibody agent, a growth hormone agent, and an insulin-like growth factor (IGF-1), however, the agent is not limited thereto.

Further, in cases where the therapeutic agent of the present invention is used in gene therapy, a therapeutic agent in which a nucleic acid encoding the amino acid sequence of an NPR-B agonist peptide downstream of a promoter sequence that functions in a host cell, for example, a cytomegalovirus promoter (CMV promoter) and the like is incorporated into a known vehicle suitable for gene therapy such as a viral vector, preferably a lentiviral vector, or an adeno-associated virus vector, and more preferably an adenovirus vector; or a chemically synthesized liposome, a viral envelope, or a complex of a viral envelope and a synthetic liposome can be used. As the gene encoding CNP, a gene containing a nucleotide sequence that encodes an amino acid sequence of any one of SEQ ID NOs: 1 to 5 may be used. As to the method of specific gene therapy, a method described in Experimental Medicine (Jikken Igaku), Vol. 12, p. 303, 1994, a method of the literature cited therein, or the like may be used.

### Examples

Hereinafter, the present invention is described in more detail by way of Examples. These examples illustrate examples of embodiments of the present invention, and the present invention is not limited to these examples.

CNP derivative (A) (SEQ ID NO: 13) and CNP derivative (C) (SEQ ID NO: 8) used in the present example were prepared according to the description of U.S. Patent Publication US 2010-305031. CNP derivative (B) (SEQ ID NO: 15) was prepared based on the description of the pamphlet of WO 2009/067639.

### <Example 1>: Preparation of dexamethasone-induced growth failure model rat

### [Method]

Osmotic pumps (MINI-OSMOTIC PUMP Model 2004, available from DURECT CORPORATION, flow rate: 0.25 µL/hr, for 28 days) were charged with dexamethasone (dexamethasone injection "KS", available from Kyoritsuseiyaku Corporation) having a concentration of 0.5 mg/mL (Group 2) or 1 mg/mL(Group 3), and one osmotic pump was embedded in the hypoderm of the back of each 7-week-old male Crl: CD (SD) rat (5 animals/group, a total of 10 animals). Additionally, in a control group (Group 1, 5 animals), one osmotic pump (the same as above) charged with saline was embedded in the hypoderm of the back of each animal. Then the animals were fed for 28 days, and their body weight was measured twice or three times a week. On the final day, after blood collection under isoflurane anesthesia followed by immediate euthanasia , the right femur was extracted, and the length was measured. Plasma was separated from the collected blood, and cholesterol concentration was determined by using cholesterol E-Test Wako (available from Wako Pure Chemical Industries, Ltd.), and the plasma corticosterone concentration was determined by using a Corticosterone, ELISA Kit, AssayMax (96 well) (EC3001-1, available from ASSAYPRO LLC.).

### [Result]

The changes in the body weight of rats during the dexamethasone administration period are shown in Fig. 1, and the length of the right femur on the final day is shown Fig. 2. By the continuous subcutaneous administration of dexamethasone, the body weight decreased dose-dependently, and the femur length shortened dose-dependently. Concentrations of corticosterone and cholesterol in the plasma collected on the final day are shown in Table 1. By the continuous subcutaneous administration of dexamethasone, the corticosterone concentration was significantly lower in Group 2 and Group 3 than in Group 1, and the concentration of cholesterol used for biosynthesis of corticosterone was significantly greater in Group 3. In the present study, the dosage of dexamethasone is 0.009 to 0.027 mg/kg/day (Table 2), and it was lower than the therapeutic dose for a dog or a cat described in the package insert of dexamethasone injection "KS" (0.05 to 0.1 mg as dexamethasone per 1 kg of body weight is subcutaneously or intramuscularly injected once a day). The study indicated that the therapeutic dose of dexamethasone induced growth failure of a rat.
[Table 1: Plasma concentrations of corticosterone and cholesterol at the end of 28-day continuous subcutaneous administration of dexamethasone to male Crl: CD (SD) rat]

**[Table 1]**

| (Mean value ± standard deviation, n = 4-5) | | | |
|---|---|---|---|
| Group | Concentrations of dexamethasone in each dosing solution | Corticosterone (ng/mL) | Cholesterol (mg/dL) |
| Group 1 | Only medium | 324.1 ± 142.7 | 61.0 ± 4.1 |
| Group 2 | 0.5 mg/mL | 32.3 ± 10.9** | 53.5 ± 7.0^{NS} |
| Group 3 | 1 mg/mL | 36.6 ± 36.3** | 78.8 ± 11.6* |

| | | | |
|---|---|---|---|
| **: Significant difference with Group 1 (p < 0.01), *: Significant difference with Group 1 (p < 0.05), NS: No significant difference with Group 1 (p > 0.05) | | | |

[Table 2: Mean dosage of dexamethasone in each group]

**[Table 2]**

| Group | Estimated dosage (mg/kg/day) | | | | | | | | |
|---|---|---|---|---|---|---|---|---|---|
| | Day 1 | Day 4 | Day 7 | Day 10 | Day 14 | Day 16 | Day 18 | Day 22 | Day 28 |
| Group 1 | - | - | - | - | - | - | - | - | - |
| Group 2 | 0.013 | 0.013 | 0.012 | 0.011 | 0.011 | 0.010 | 0.010 | 0.010 | 0.009 |
| Group 3 | 0.027 | 0.026 | 0.024 | 0.023 | 0.023 | 0.022 | 0.022 | 0.022 | 0.021 |

| | | | | | | | | | |
|---|---|---|---|---|---|---|---|---|---|
| -: Only medium | | | | | | | | | |

### <Example 2> Combined administration study of CNP derivative (A) and dexamethasone which is a growth failure inducer in rats

### [Method]

Osmotic pumps (MINI-OSMOTIC PUMP Model 2004, available from DURECT CORPORATION, flow rate: 0.25 µL/hr, for 28 days) were charged with a 1 mg/mL solution of dexamethasone (dexamethasone injection "KS", available from Kyoritsuseiyaku Corporation, Group 2 and Group 3) or saline in a control group (Group 1), and one osmotic pump was embedded in the hypoderm of the back of each 7-week-old male Crlj : WI (Wistar) rat. At the same time, one osmotic pump (the same as described above) charged with a 20 mg/mL solution of CNP derivative (A) in Group 3, or charged with Medium 1 (0.03 mol/L acetate buffer (pH 4.0)/1% benzyl alcohol/10% sucrose) in Group 1 and Group 2 was embedded in the hypoderm of the back simultaneously with the pump containing dexamethasone or saline. Then the animals were fed for 28 days, and the body weight, the body length (length from the top of the nose to the anus) and the tail length were measured on the final day, and after collecting the blood from the abdominal aorta under isoflurane anesthesia, the right femur and major organs (spleen, thymus, liver, heart and lung) were collected. Regarding the femur, the bone length and width were measured by using a caliper, and the organ weights were measured. Concentrations of cholesterol and IGF-1 in plasma prepared from the blood, were determined by using cholesterol E- Test Wako (available from Wako Pure Chemical Industries, Ltd.) and Quantikine(R) ELISA Mouse/Rat IGF-I Immunoassay (MG100, available from R&D Systems).

### [Result]

The body weight, the body length and the tail length after continuous subcutaneous administration of dexamethasone and CNP derivative (A) for 28 days are shown in Table 3, and the length and width of the femur are shown in Table 4. The body weight on the final day was significantly lower in Group 2 and Group 3 than in Group 1, (p < 0.01), and the effect of combined administration of CNP derivative (A) was not observed. On the other hand, the body length, the tail length and the femur length were significantly shortened by dexamethasone treatment(Group 2) (p < 0.01), and recovered to the same level as in Group 1 by the combined administration of CNP derivative (A) (Group 3) . Regarding the femur width, although the tendency of shortening by the administration of dexamethasone was observed, this was not significant (p > 0.05), and the influence of the combined use of CNP derivative (A) was not determined. Cholesterol and IGF-1 concentration on the final day are shown in Table 5. Cholesterol concentration in the plasma on the final day significantly increased due to the administration of dexamethasone (Group 2) (p < 0.01). Due to a large variation in data within Group 3, no significant difference was observed between Group 3 and either of Group 1 or 2. The plasma IGF-1 concentration significantly decreased in Group 2 and 3, compared with Group 1 (p < 0.05).

The weights of major organs on the final day are shown in Table 6. The weights of spleen and thymus administered with dexamethasone in Group 2 and Group 3 (body weight ratio (%)) were significantly lower than Group 1, and the influence of the combined administration of CNP derivative (A) was not observed. Therefore, it was considered that the immunosuppressive effect of dexamethasone could not be inhibited by the combined administration of CNP derivative (A).

As described above, it was revealed that bone elongation suppression induced by the administration of dexamethasone (Groups 2 and 3) was evidently ameliorated by the combined administration of CNP derivative (Group 3). On the other hand, when dexamethasone was administered to a rat, symptoms such as body weight loss, increase in plasma cholesterol concentration, decrease in IGF-1 concentration, and reduction in weights of spleen and thymus were observed, but no influence by combined administration of CNP derivative (A) on these symptoms was observed.
[Table 3: Body weight, body length and tail length after 28-day continuous subcutaneous administration of dexamethasone and CNP derivative (A) to male Crlj: WI (Wistar) rat]

**[Table 3]**

| (Mean value ± standard deviation, n = 6) | | | | | |
|---|---|---|---|---|---|
| Group | Concentration of dexamethasone in each dosing solution | Concentration of CNP derivative(A) in each dosing solution | At final day | | |
| | | | Body weight (g) | Body length (mm) | Tail length (mm) |
| Group 1 | Saline | Vehicle 1 | 445 ± 28 | 233 ± 5 | 210 ± 7 |
| Group 2 | 1 mg/mL | Vehicle 1 | 352 ± 24 ** | 219 ± 5** | 195 ± 6** |
| Group 3 | 1 mg/mL | 20 mg/mL | 344 ± 34** | 233 ± 3^{NS} | 211 ± 10 ^{NS} |

| | | | | | |
|---|---|---|---|---|---|
| Vehicle 1: 0.03 mol/L acetate buffer (pH 4.0)/1% benzyl alcohol/10% sucrose **: Significant difference with Group 1 (p < 0.01), NS: No significant difference with Group 1 (p > 0.05), | | | | | |

[Table 4: Length and thickness of right femur after 28-day continuous subcutaneous administration of dexamethasone and CNP derivative (A) to male Crlj: WI (Wistar) rat]

**[Table 4]**

| (Mean value ± standard deviation, n=6) | | |
|---|---|---|
| Group | Right femur | |
| | Length (mm) | Wide (mm) |
| Group 1 | 38.1 ± 0.4 | 8.1 ± 0.3 |
| Group 2 | 35.6 ± 0.6 ** | 7.7 ± 0.4 ^{NS} |
| Group 3 | 37.9 ± 0.6 ^{NS} | 7.8 ± 0.2 ^{NS} |

| | | |
|---|---|---|
| Wide is indicated for epiphyseal region (maximum width) **: Significant difference with Group 1 (p < 0.01), NS: No significant difference with Group 1 (p > 0.05) | | |

[Table 5: Plasma concentrations of cholesterol and IGF-1 after 28-day continuous subcutaneous administration of dexamethasone and CNP derivative (A) to male Crlj : WI (Wistar) rat]

**[Table 5]**

| (Mean value ± standard deviation, n = 5-6) | | |
|---|---|---|
| Group | Plasma concentration | |
| | Cholesterol (mg/dL) | IGF-1 (ng/mL) |
| Group 1 | 69.7 ± 9.1 | 1452 ± 133 |
| Group 2 | 85.3 ± 5.9** | 1207 ± 195* |
| Group 3 | 77.0 ± 13.3^{NS} | 1207 ± 132* |

| | | |
|---|---|---|
| **: Significant difference with Group 1 (p < 0.01), *: Significant difference with Group 1 (p < 0.05), NS: No significant difference with Group 1 (p > 0.05) | | |

[Table 6: Weights of major organs after 28-day continuous subcutaneous administration of dexamethasone and CNP derivative (A) to male Crlj: WI (Wistar) rat]

**[Table 6]**

| (Mean value ± standard deviation, n = 6) | | | | | |
|---|---|---|---|---|---|
| Group | Upper stage: Organ weight (g) [Lower stage: Body-weight ratio (%)] | | | | |
| | Spleen | Thymus | Liver | Heart | Lung |
| Group 1 | 1.29 ± 0.22 [0.29 ± 0.04%] | 0.45 ± 0.09 [0.10 ± 0.02%] | 16.67 ± 1.29 [3.75 ± 0.22%] | 1.19 ± 0.10 [0.27 ± 0.02%] | 1.57 ± 0.10 [0.35 ± 0.03%] |
| Group 2 | 0.79 + 0.09 [0.22 ± 0.04%*] | 0.21 ± 0.05 [0.06 ± 0.01%**] | 14.48 ± 2.37 [3.80 ± 0.31% ^{NS}] | 1.05 ± 0.09 [0.29 ± 0.02% ^{NS}] | 1.29 + 0.09 [0.36 ± 0.01 % ^{NS}] |
| Group 3 | 0.76 ± 0.08 [0.23 ± 0.03%*] | 0.17 ± 0.04 [0.05 ± 0.01%**] | 15.03 ± 5.04 [3.91 ± 0.20% ^{NS}] | 0.93 ± 0.06 [0.28 ± 0.02% ^{NS}] | 1.26 ± 0.10 [0.37 ± 0.01% ^{NS}] |

| | | | | | |
|---|---|---|---|---|---|
| **: Significant difference with Group 1 (p < 0.01), *: Significant difference with Group 1 (p < 0.05), NS: No significant difference with Group 1 (p > 0.05) | | | | | |

### <Example 3> Combined administration study of human-type CNP-53 or various CNP derivatives in dexamethasone-induced rat growth failure model

### [Method]

Osmotic pumps (MINI-OSMOTIC PUMP Model 2004, available from DURECT CORPORATION, flow rate: 0.25 µL/hr, for 28 days) were charged with 0.67 mg/mL solution of dexamethasone (dexamethasone injection "KS", available from Kyoritsuseiyaku Corporation) and one osmotic pump was embedded in the hypoderm of the back of each 5-week-old male Crlj: WI (Wistar) rat (5 animals/group, Groups 2 to 6, a total of 25 animals). At the same time, one osmotic pump (the same as above) charged with a 15 mg/mL solution of CNP derivative (A) in Group 3, human-type CNP-53 in Group 4, CNP derivative (B) in Group 5 and CNP derivative (C) in Group 6 was embedded in the hypoderm of the back simultaneously. No substance was administered to the normal control group (Group 1, 5 animals). Then the animals were fed for 28 days, and transitions of the body weight, the body length and the tail length were measured. On the final day, the blood was collected from the abdominal aorta and the animals were caused to die under isoflurane anesthesia, and plasma was separated from the collected blood and the cholesterol concentration was determined by using a "LabAssay (trademark) cholesterol" (available from Wako Pure Chemical Industries, Ltd.). After collecting the blood and causing the animals to die, the right femur and thymus were extracted, and the length of the right femur and the weight of the thymus were measured.

### [Result]

The body weight, the body length, the tail length and the femur length on the final day when either one of human-type CNP-53, CNP derivative (A), (B) or (C) was used in 28-day continuous subcutaneous administration in simultaneous combination with continuous subcutaneous administration of dexamethasone are shown in Table 7. In the dexamethasone administration group (Group 2), increase in the body weight of a rat, and elongation of the body and the tail length were significantly suppressed, and in groups in which either one of human-type CNP-53, or CNP derivative (A), (B) or (C) was administered in combination (Group 3 to Group 6), elongation of the body length and the tail length was significantly promoted in comparison with the single administration group of dexamethasone (Group 2) (p < 0.05 or 0.01). The femur length significantly shortened due to the administration of dexamethasone, and the femur length showed the tendency of being longer in groups in which either one of human-type CNP-53, or CNP derivative (A), (B) or (C) was administered in combination (Group 3 to Group 6) rather than in the single administration group of dexamethasone (Group 2). On the other hand, a clear difference was not observed in the plasma cholesterol concentration and the thymus weight between the single administration group of dexamethasone (Group 2) and groups in which either one of human-type CNP-53, or CNP derivative (A), (B) or (C) was administered in combination (Group 3 to Group 6) (Table 8).
[Table 7: Body weight, body length, tail length and right femur length after the 28-day continuous subcutaneous administration of dexamethasone and various CNP derivatives to 5-week-old male
Crlj: WI (Wistar) rats]

**[Table 7]**

| (Mean value ± standard deviation, n = 5) | | | | | | | | | |
|---|---|---|---|---|---|---|---|---|---|
| Group | | Upper stage: at dissection (the day after final day of 28-day administration) | | | | | | | |
| | | Lower stage in parentheses: variation from preadministration value (Δ value) | | | | | | | |
| | | Body weight (g) | | Body length (mm) | | Tail length (mm) | | Right femur length (mm) | |
| Group 1 | Normal control | 389 ± 27 | - | 219 ± 6 | - | 224 ± 8 | - | 36.5 ± 0.7 | - |
| | | (215 ± 24) | - ** | (44.6 ± 5.4) | -** | (58.0 ± 4.5 ) | -** | | ** |
| Group 2 | Dexamethasone | 286 ± 17 | ## | 199 ± 2 | ## | 202 ± 3 | ## | 32.7 ± 0.3 - | ## |
| | | (118 ± 15) | ## - | (23.8 ± 2.4) | ##- | (35.2 ± 3.7) | ##- | | - |
| Group 3 | Dexamethasone + CNP derivative (A) | 307 ± 27 | ## | 212 ± 12 | NS | 216 ± 13 | NS | 34.1 ± 1.5 | # |
| | | (136 ± 24) | ## NS | (36.8 ± 10.4) | NS * | (48.4 ± 11.4) | NS * | | NS |
| Group 4 | Dexamethasone + human-type CNP-53 | 302 ± 7 | ## | 208 ± 4 | ## | 210 ± 4 | ## | 34.1 ± 1.3 | ## |
| | | (130 ± 12) | ## NS | (34.4 ± 6.0) | # ** | (44.6 ± 7.5) | ##* | | * |
| Group 5 | Dexamethasone + CNP derivative (B) | 304 ± 23 | ## | 210 ± 10 | NS | 205 ± 6 | ## | 34.1 ± 1.7 | # |
| | | (133 ± 18) | ## NS | (36.2 ± 9.3) | NS* | (41.6 ± 4.3) | ##* | | NS |
| Group 6 | Dexamethasone + CNP derivative (C) | 305 ± 12 | ## | 208 ± 6 | # | 205 ± 1 | ## | 34.1 ± 0.2 | ## |
| | | (135 ± 11) | ## NS | (34.2 ± 7.4) | #* | (41.6 ± 1.9) | ##** | | ** |

| | | | | | | | | | |
|---|---|---|---|---|---|---|---|---|---|
| With Group 1, ##: Significant difference (p < 0.01), #: Significant difference (p < 0.05), NS: No significant difference (p > 0.05) With Group 2, **: Significant difference (p < 0.01), *: Significant difference (p < 0.05), NS: No significant difference (p > 0.05) | | | | | | | | | |

[Table 8: Plasma cholesterol concentration and thymus weight after the 28-day continuous subcutaneous administration of dexamethasone and various CNP derivatives to 5-week-old male Crlj : WI (Wistar) rats]

**[Table 8]**

| (Mean value ± standard deviation, n = 5) | | | |
|---|---|---|---|
| Group | | Plasma cholesterol concentration (mg/dL) | Upper stage: thymus weight at dissection (g) [Lower stage: body weight ratio (%)] |
| Group 1 | Normal control | 55.8 ± 3.8** | 0.77 ± 0.08 [0.20 ± 0.01%**] |
| Group 2 | Dexamethasone | 65.1 ± 5.9 | 0.36 ± 0.04 [0.11 ± 0.02%] |
| Group 3 | Dexamethasone + CNP derivative (A) | 61.3 ± 3.0 ^{NS} | 0.41 ± 0.15 [0.13 ± 0.04% ^{NS}] |
| Group 4 | Dexamethasone + human-type CNP-53 | 62.1 ± 2.2 ^{NS} | 0.33 ± 0.03 [0.11 ± 0.01% ^{NS}] |
| Group 5 | Dexamethasone + CNP derivative (B) | 64.4 ± 0.8 ^{NS} | 0.42 ± 0.14 [0.14 ± 0.04%*] |
| Group 6 | Dexamethasone + CNP derivative (C) | 59.2 ± 7.4 ^{NS} | 0.35 ± 0.06 [0.11 ± 0.02% ^{NS}] |

| | | | |
|---|---|---|---|
| **: Significant difference with Group 2 (p < 0.01), *: Significant difference with Group 2 (p < 0.05), NS: No significant difference with Group 2 (p > 0.05) | | | |

### <Example 4> Follow-up administration test of CNP derivative (A) for rat growth failure induced by dexamethasone

### [Method]

Osmotic pumps (MINI-OSMOTIC PUMP Model 2004, available from DURECT CORPORATION, flow rate: 0.25 µL/hr, for 28 days) were charged with a 0.67 mg/mL solution of dexamethasone (dexamethasone injection "KS", available from Kyoritsuseiyaku Corporation), and one osmotic pump was embedded in the hypoderm of the back of each 4-week-old male Crlj: WI (Wistar) rat (5 animals/group, Group 2 and Group 3), and the animals were fed for 14 days. After confirming significant reduction (reducing the body weight or shortening the body length or the tail length) due to the administration of dexamethasone, an osmotic pump (the same as described above) charged with a 15 mg/mL solution of CNP derivative (A) was additionally embedded in the hypoderm of the back of only the animals in Group 3, and the animals were fed for another 28 days . During the feeding period, transitions of the body weight, the body length and the tail length were measured, and on the final day, blood was collected from the abdominal aorta and the animal was caused to die under isoflurane anesthesia, the right femur was extracted and its length was measured.

### [Result]

The transitions of the body weight, the body length and the tail length in rats during the test period are shown in Fig. 3. After 14 days from starting administration of dexamethasone, the increase in the body weight of the rats, and elongation of the body and the tail length were significantly suppressed in Group 2 in comparison with Group 1 (Table 9). Then, administration of CNP derivative (A) was started only in Group 3, and elongation in the body length and the tail length increased in comparison with the non-administered group (Group 2), and the body length and the tail length after 28 days from starting administration of CNP derivative (A) was significantly longer in Group 3 than in Group 2 (p < 0.01). Also the femur after 28 days from starting administration of CNP derivative (A) was significantly longer than in the non-administered group (p < 0.01) (Fig. 4), but a significant change in the transition of the body weight by the combined administration of CNP derivative (A) was not observed.
[Table 9: Body weight, body length, tail length and femur length when CNP derivative (A) was subcutaneously administered continuously for 28 days to male Crlj : WI (Wistar) rats in which growth failure was induced by continuous subcutaneous administration of dexamethasone]

**[Table 9]**

| (Mean value ± standard deviation, n = 5) | | | | | |
|---|---|---|---|---|---|
| Group | | Upper stage: Day 14 of dexamethasone administration (prior to administration of CNP derivative (A)) | | | |
| | | Middle stage: at dissection (Day 28 of CNP derivative (A) administration) | | | |
| | | Lower stage: in parentheses: variation (Δ value) | | | |
| | | Body weight (g) | Body length (mm) | Tail length (mm) | Right femur length (mm) only at dissection |
| Group 1 | Normal control | 229 ± 8** | 195 ± 1** | 188 ± 6** | 36.7 ± 0.4** |
| | | 405 ± 8** | 227 ± 5** | 229 ± 6** | |
| | | (176 ± 4**) | (32 ± 4**) | (41 ± 3) | |
| Group 2 | Dexamethasone | 179 ± 12 | 174 ± 4 | 177 ± 7 | 32.9 ± 0.7 |
| | | 308 ± 20 | 204 ± 3 | 202 ± 8 | |
| | | (129 ± 9) | (30 ± 1) | (25 ± 7) | |
| Group 3 | Dexamethasone + CNP derivative (A) | 179 ± 15 ^{NS} | 175 ± 5 ^{NS} | 172 ± 6 ^{NS} | 36.4 ± 0.2** |
| | | 333 ± 23 ^{NS} | 223 ± 6** | 219 ± 5 ** | |
| | | (154 ± 9**) | (47 ± 5**) | (47 ± 6**) | |

| | | | | | |
|---|---|---|---|---|---|
| In parentheses: increment from starting of administration of CNP derivative (A) to Group 3 **: Significant difference with Group 2 (p < 0.01), *: Significant difference with Group 2 (p < 0.05), NS: No significant difference with Group 2 (p > 0.05) | | | | | |

### <Example 5> Simultaneous combined administration test of various steroids and CNP derivative (A) in rats

### [Method]

0.3 mg/mL solution of dexamethasone (dexamethasone injection "KS", available from Kyoritsuseiyaku Corporation diluted with saline), prednisolone succinate suspended in 0.1% polysorbate-containing brine (available from Wako Pure Chemical Industries, Ltd., concentration of 5 mg/mL as prednisolone), or betamethasone dipropionate suspended in 0.1% polysorbate-containing brine (available from Wako Pure Chemical Industries, Ltd., concentration of 0.3 mg/mL as betamethasone) was administered to the hypoderm of the back of a 4-week-old male Crlj : WI (Wistar) rat (ten animals for each set of substances) in a volume of 1 mL/kg once a day for 28 days. No substance was administered to the control group (5 animals, Group 1). At the same time, for five of ten animals in each set (Group 2, Group 4, Group 6) a medium (0.03 mol/L acetate buffer (pH 4.0)/1% benzyl alcohol/10% sucrose) and for the remaining five animals (Group 3, Group 5, Group 7) the medium and a 1 mg/mL solution of CNP derivative (A) were administered in combination in the hypoderm of the back in a volume of 1 mL/kg once a day for 28 days. After measuring the body weight, the body length and the tail length under isoflurane anesthesia on the day after the final day of administration, blood was collected from the abdominal aorta and the animal was caused to die, the right femur was extracted and the femur length was measured.

### [Result]

Since significant suppression of body weight gain of rats, and significant deterioration in: length of the body; length of the tail; and length growth of the femur were observed with repeated administration for 28 days (p < 0.05 or 0.01) (Table 10) of any of dexamethasone, prednisolone and betamethasone, these steroids are considered to induce growth failure in rats. Combined repeated subcutaneous administration of CNP derivative (A) in a dose of 1 mg/kg conducted simultaneously with the repeated administration of these steroids resulted in significant elongation of the body length, the tail length and the femur in comparison with the non-administered group of CNP derivative (A) (p < 0.05 or 0.01). These reveal that CNP derivative (A) reduces and/or ameliorates growth failure induced by the administration of other steroids such as prednisolone and betamethasone as well as dexamethasone. [Table 10: Body weight, body length, tail length and right femur length when dexamethasone, prednisolone or betamethasone was subcutaneously administered to male Crlj : WI (Wistar) rats once a day repeatedly for 28 days]

**[Table 10]**

| (Mean value ± standard deviation, n = 5) | | | | | | | | | |
|---|---|---|---|---|---|---|---|---|---|
| Group | | Upper stage: at dissection (The day after final day of 28-day administration of CNP derivative (A)) | | | | | | | |
| | | Lower stage in parentheses: variation from preadministration value (Δ value) | | | | | | | |
| | | Body weight (g) | | Body length (mm) | | Tail length (mm) | | Right femur length (mm) | |
| Group 1 | Normal control | 356 ± 18 | - | 218 ± 6 | - | 218 ± 2 | - | 34.9 ± 0.5 | -- |
| | | (250 ± 17) | -- | (68 ± 6) | -- | (92 ± 7) | -- | | |
| Group 2 | Dexamethasone + Medium | 176 ± 5 | ## | 177 ± 4 | ## | 174 ± 4 | ## | 28.3 ± 0.4 | ##- |
| | | (69 ± 5) | ##- | (27 ± 5) | ##- | (47 ± 7) | ##- | | |
| Group 3 | Dexamethasone + CNP derivative (A) | 187 ± 10 | ## | 189 ± 3 | ## | 184 ± 4 | ## | 29.6 ± 0.3 | ##** |
| | | (81 ± 8) | ##* | (39 ± 3) | ##** | (56 ± 3) | ##* | | |
| Group 4 | Prednisolone + Medium | 298 ± 14 | ## | 209 ± 4 | # | 206 ± 6 | ## | 33.3 ± 0.3 | ##- |
| | | (191 ± 14) | ## | (58 ± 4) | #- | (79 ± 9) | # - | | |
| Group 5 | Prednisolone + CNP derivative (A) | 315 ± 21 | # | 221 ± 3 | NS | 225 ± 7 | ND | 34.2 ± 0.5 | NS |
| | | (208 ± 17) | ##NS | (71 ± 4) | NS ** | (97 ± 5) | NS ** | | |
| Group 6 | Betamethasone + Medium | 315 ± 11 | ## | 211 ± 2 | # | 213 ± 4 | # | 33.9 ± 0.5 | #- |
| | | (208 ± 10) | ##- | (59 ± 5) | # - | (82 + 3) | #- | | |
| Group 7 | Betamethasone + CNP derivative (A) | 331 ± 20 | NS | 230 ± 4 | ## | 229 ± 1 | ## | 35.6 ± 0.5 | NS ** |
| | | (224 ± 18) | #NS | (78 ± 6) | # ** | (101 ± 4) | # ** | | |

| | | | | | | | | | |
|---|---|---|---|---|---|---|---|---|---|
| With Group 1, ##: Significant difference (p < 0.01), #: Significant difference (p < 0.05), NS: No significant difference (p > 0.05) In significant difference test of Group 2 vs Group 3, Group 4 vs Group 5, Group 6 vs Group 7, with Group 2, Group 4, or Group 6, **: Significant difference (p < 0.01), *: Significant difference (p < 0.05), NS: No significant difference (p > 0.05) | | | | | | | | | |

### <Example 6> Action comparing study between CNP derivative (A) and human growth hormone on rat growth failure induced by dexamethasone

### [Method]

Osmotic pumps (MINI-OSMOTIC PUMP Model 2004, available from DURECT CORPORATION, flow rate: 0.25 µL/hr, for 28 days) were charged with a 0.67 mg/mL solution of dexamethasone (dexamethasone injection "KS", available from Kyoritsuseiyaku Corporation), and one osmotic pump was embedded in the hypoderm of the back of each 4-week-old male Crlj: WI (Wistar) rat (5 animals/group, Group 2 to Group 4, total of 15 animals). For Group 3, a 1 mg/mL solution of CNP derivative (A) (medium: 0.03 mol/L acetate buffer (pH 4.0)/1% benzyl alcohol /10% sucrose), and for Group 4, a 1 mg/mL solution of human-type growth hormone preparation (somatropin BS subcutaneous injection 10 mg "SANDOZ") diluted with a phosphate buffer was administered to the hypoderm of the back of five rats, respectively, in a volume of 2 mL/kg once a day for 28 days. No substance was administered to the normal control group (5 animals, Group 1). On the final day, after measuring the body weight, the body length and the tail length, the blood was collected from the abdominal aorta and the animal was caused to die under isoflurane anesthesia, the left femur was extracted and the bone length was measured, and then a histopathological specimen of the epiphyseal region was prepared.

### [Result]

The body weight, the body length, the tail length and the left femur length when dexamethasone and CNP derivative (A) or human-type growth hormone were administered in combination are shown in Table 11. In the dexamethasone-administration group (Group 2), body weight gain, and elongation of the body length and the tail length were significantly suppressed (p < 0.01). In the combined administration group of CNP derivative (A) (Group 3), the elongation impairment of the body and the tail length were significantly ameliorated in comparison with the dexamethasone-only administration group (Group 2) (p < 0.01 and p < 0.05). In the combined administration group of human-type growth hormone (Group 4), no significant difference in elongation of the body length and the tail length was observed in comparison with the dexamethasone-only administration group (Group 2) (p > 0.05). The left femur length on the final day was significantly shorter due to administration of dexamethasone (Group 2) in comparison with Group 1 (p < 0.01). The left femur length on the final day was significantly longer in the combined administration group of CNP derivative (A) (Group 3) in comparison with the dexamethasone-only administration group (Group 2) (p < 0.01). No significant difference was observed between the combined administration group of human-type growth hormone (Group 4) and the dexamethasone-only administration group (Group 2) (p > 0.05). The growth plate cartilage layer of the epiphyseal region of the left femur exhibited the tendency of narrowing due to the administration of dexamethasone only, and swelling by combined administration of CNP derivative (A) or human-type growth hormone in comparison with the administration of dexamethasone only (Fig. 5).
[Table 11: Body weight, body length, tail length and right femur length after the 28-day continuous subcutaneous administration of dexamethasone to 4-week-old male Crlj : WI (Wistar) rats, and influence of combined repeated subcutaneous administration of CNP derivative (A) or human-type growth hormone.

**[Table 11]**

| (Mean value ± standard deviation, n = 4 or 5) | | | | | | | | | |
|---|---|---|---|---|---|---|---|---|---|
| Group | | Upper stage: at dissection (the day after final day of 28-day administration) | | | | | | | |
| | | Lower stage in parentheses: variation from pre-administration value (Δ value) | | | | | | | |
| | | Body weight (g) | | Body length (mm) | | Tail length (mm) | | Left femur length (mm) | |
| Group 1 | Normal control group | 339 ± 24 | - | 219 ± 3 | - | 218 ± 6 | - | 33.8 ± 0.4 | - |
| | | (232 ± 25) | - ** | (66.8 ± 3.4) | - ** | (85.8 ± 3.4) | -** | | ** |
| Group 2 | Dexamethasone only administration | 245 ± 12 | ## | 199 ± 4 | ## | 197 ± 5 | ## | 31.3 ± 0.2 | ## |
| | | (139 ± 13) | ## - | (46.8 ± 4.7) | ## - | (65.0 ± 4.9) | ##- | | - |
| Group 3 | Dexamethasone + CNP derivative (A)¹⁾ | 250 ± 20 | ## | 210 ± 5 | ## | 209 ± 2 | # | 32.7 ± 0.4 | ## |
| | | (145 ± 15) | ## NS | (56.3 ± 2.9) | ## ** | (73.5 ± 1.3) | ## * | | ** |
| Group 4 | Dexamethasone + human-type growth hormone | 255 ± 7 | ## | 199 ± 5 | ## | 199 ± 3 | ## | 31.7 ± 0.6 | ## |
| | | (149 + 3) | ## NS | (44.8 ± 3.7) | ## NS | (66.8 ± 5.0) | ## NS | | NS |

| | | | | | | | | | |
|---|---|---|---|---|---|---|---|---|---|
| 1) The number of cases was four because of one case in which the pump charged with dexamethasone dropped off from the hypoderm during the test and so was removed. With Group 1: ##: Significant difference (p < 0.01), #: Significant difference (p < 0.05), NS: No significant difference (p > 0.05) With Group 2; ##: Significant difference (p < 0.01), *: Significant difference (p < 0.05), NS: No significant difference (p > 0.05) | | | | | | | | | |

## Claims

1. A medicine for reducing and/or ameliorating growth failure induced by the administration of a steroid, the medicine comprising at least one natriuretic peptide receptor B (NPR-B) agonist as an active ingredient, wherein the medicine is administered to an individual in a growth period, the individual being in need of continuous or continual administration of a steroid.

2. The medicine according to claim 1, wherein the NPR-B agonist is C-type natriuretic peptide (CNP), an active fragment thereof, a mutant thereof, a derivative thereof or a modification thereof, or an anti-NPR-B antibody.

3. The medicine according to claim 1, wherein the NPR-B agonist is hCNP-22 (SEQ ID NO: 1), hCNP6-22 (amino acid Nos. 6 to 22 in SEQ ID NO: 1) or hCNP-53 (SEQ ID NO: 2), a mutant thereof, a derivative thereof or a modification thereof.

4. The medicine according to claim 1, wherein the NPR-B agonist is a derivative or a modification of hCNP-22 or hCNP6-22.

5. The medicine according to any one of claims 2 to 4, wherein the derivative is a peptide in which at least one additional peptide selected from a peptide derived from an Fc region of immunoglobulin, serum albumin, and a partial peptide from the C-terminal end of ghrelin, is fused to one or both of the N terminus and the C terminus of hCNP-22 or hCNP6-22.

6. The medicine according to any one of claims 2 to 4, wherein the derivative is a peptide having an amino acid sequence selected from SEQ ID NOs: 6 to 15.

7. The medicine according to claim 6, wherein the derivative is a peptide having an amino acid sequence of SEQ ID NO: 8, 13 or 15.

8. The medicine according to any one of claims 2 to 4, wherein the modification is such that PEG or a related hydrophilic polymer is conjugated to one or both of the N terminus and the C terminus of hCNP-22, hCNP6-22, or hCNP-53.

9. The medicine according to any one of claims 1 to 8, wherein it is administered at the start of, or during the period of, a steroid therapy, to reduce and/or ameliorate growth failure.

10. The medicine according to any one of claims 1 to 8, wherein it is administered to treat dwarfism during or after the end of a steroid therapy period, the dwarfism having occurred due to growth failure induced by the administration of the steroid.
